# EUROPÄISCHE PATENTSCHRIFT

(11) **EP 0 391 384 B1**
(45) Veröffentlichungstag und Bekanntmachung des Hinweises auf die Patenterteilung: **31.08.1994**
(21) Anmeldenummer: 90106422.0
(22) Anmeldetag: 04.04.1990
(51) Int. Cl.: A61B 17/22

(54) **Lithotriptor**
Lithotriptor
Lithotriteur

(30) Priorität: 05.04.1989 DE 8904213 U; 05.04.1989 DE 8904212 U
(43) Veröffentlichungstag der Anmeldung: 10.10.1990
(73) Patentinhaber: GIP Medizin Technik GmbH, 83224 Grassau (DE)
(72) Erfinder: Wechler, Ingolf Max, D 8221 Grabenstätt (DE)
(74) Vertreter: Viering, Jentschura & Partner

(56) Entgegenhaltungen:
- EP-A- 0 152 032
- DE-A- 3 521 717
- DE-U- 8 501 297
- FR-A- 2 317 903

## Beschreibung

Die Erfindung betrifft einen Lithotriptor mit einem Fangkörbchen, das von mehreren nach außen gebogen verlaufenden Fangsträngen aus Drahtmaterial gebildet wird und einen Fangkörbchenfuß sowie eine Fangkörbchenspitze aufweist, an der die Fangstränge aneinander befestigt sind, die an dem Fangkörbchenfuß mit einem aus mehreren Zugdrahten bestehenden Zugstrang verbunden sind, welcher über einen verseilten Längenbereich als verseiltes Drahtseil ausgebildet ist, in einer Ummantelung geführt ist und von einer Zugvorrichtung zum Ein- und Ausfahren des Fangkörbchens in der Ummantelung verschiebbar ist wobei die Litzenzahl der Drahtseils mindestens der Anzahl der Fangstränge des Fangkörbchens entspricht, die von verformten Seillitzen des Drahtseils gebildet werden, welche vom Drahtseil aus bis zur Fangkörbchenspitze durchlaufen.

Ein herkömmlicher Lithotriptor ist beispielsweise aus dem DE-GM 87 06 587 bekannt. Dort ist ein endoskopischer Lithotriptor mit einem flexiblen Katheter beschrieben, in dessen distales Ende ein aus mehreren Fangdrähten gebildetes Fangkörbchen mittels eines mit den Fangdrähten verbundenen, durch den Katheter geführten Zugstranges einfahrbar ist, welcher an einer Zugstange festgelegt ist, die in einem Griffstück, an welchem der Katheter befestigt ist, axial verschiebbar ist und ein Außengewinde aufweist, welches in eine an dem Griffstück abstützbare Stellmutter eingreift. Ein solcher Lithotriptor wird durch einen Instrumentierkanal eines Endoskops in einen Körperkanal eingeführt und dient dazu, größere Steine und sonstige größere Konkremente noch im Körper des Patienten zu zerkleinern. Hierzu wird der Katheter bei in dessen distales Ende eingefahrenem Fangkörbchen über den Instrumentierkanal des Endoskops eingeführt, bis das distale Ende des Katheters aus dem Instrumentierkanal des Endoskops austritt. Dann wird die Zugstange in das Griffstück eingeschoben, wodurch das Fangkörbchen aus dem distalen Ende des Katheters austritt und sich aufgrund der Verformung der Fangdrähte entfaltet. Nachdem der Stein in dem Fangkörbchen eingefangen ist, wird durch Herausziehen der Zugstange aus dem Griffstück das Fangkörbchen soweit in den Katheter eingefahren, bis die Fangdrähte den Stein fest umschlossen haben. Danach wird die Stellmutter auf dem Außengewinde der Zugstange bis zum Anschlagen an dem zugeordneten Stirnende des Griffstückes verschraubt, wonach durch weiteres Drehen an der Stellmutter die Fangdrähte des Fangkörbchens mit verhältnismäßig großer Kraft, die aufgrund der Gewindeübersetzung erzeugt wird, weiter in das distale Ende des Katheters hineingezogen werden, sich in den Stein einschneiden und diesen zerlegen. Hierzu müssen meistens erhebliche Zugkräfte auf das Fangkörbchen zum Zerlegen auch harter Konkremente übertragen werden. Der Zugstrang muß hierzu eine dementsprechende Zugfestigkeit aufweisen. Darüberhinaus muß der Zugstrang ausreichend fest in der Klemmvorrichtung festgelegt werden und darf in seiner Festigkeit durch die Klemmvorrichtung selbst nicht geschwächt werden. Bei diesem Lithotriptor erfolgt das Festklemmen des Zugstrangs mittels einer Klemmschraube, was bei sehr starkem seitlichen Klemmdruck zu einer Beschädigung des Zugstranges führen kann.

Ein weiterer Lithotriptor ist aus dem DE-GM 86 26 048 bekannt. Dort wird das Fangkörbchen aus drei endlos schlaufenförmigen Fangsträngen aus jeweils mehreren geflochtenen oder gedrehten Einzeldrähten gebildet. Durch das Herstellen der Fangstränge aus jeweils mehreren geflochtenen oder gedrehten Einzeldrähten wird zwar die Zugfestigkeit der einzelnen Fangstränge gesteigert, jedoch laufen diese Fangstränge am Fuß des Fangkörbchens ohne Unterbrechung in den Zugstrang ein, der somit aus mehreren parallelen Einzelsträngen gebildet wird, die bis zum bedienungsseitigen Geräteende hin durchlaufen. Ein derartiger Zugstrang aus mehreren Einzelsträngen führt jedoch zu Problemen bei der Festlegung des Zugstranges im bedienungsseiten Griffstück des Lithotriptors bzw. in der im Griffstück ausgebildeten Zugvorrichtung zum Anziehen des Zugstranges und zu Problemen bei der Verteilung der Kräfte über die Einzelstränge hin. So kann es dazu kommen, daß bei dem Versuch der Zerlegung eines in dem Fangkörbchen eingefangenen, sehr harten Steins ein Teil der Einzelstränge überlastet wird und reißt, was in der Folge zum Abreißen auch der anderen Einzelstränge führen kann, so daß das Fangkörbchen und die abgerissenen Strangteile nur noch operativ entfernt werden können. Darüberhinaus besteht bei diesem bekannten Lithotriptor die Gefahr, daß das Fangkörbchen nicht von einem sehr harten und daher unzertrümmerbaren Konkrement gelöst werden kann und dieses zu groß ist, als daß es mit dem Fangkörbchen durch den Arbeitskanal des Endoskopes herausgezogen werden könnte. Auch in einem solchen Fall würde nur noch ein operativer Eingriff zum Entfernen des Lithotriptors aus dem Körper des Patienten helfen.

Ein weiterer Lithotriptor mit den Merkmalen im Oberbegriff des Patentanspruchs 1 ist aus der FR-A-2 317 903 bekannt, gemäß welcher der Zugstrang über einen verseilten Längenbereich als verseiltes Drahtseil ausgebildet ist. Das proximale Ende dieses Drahtseils ist in einen Handgriff unlösbar eingeschweißt oder eingeklebt. Die verformten Seillitzen sind im Abstand von dem distalen Ende des Drahtseils ausgebildet, so daß sie von der Fangkörbchenspitze aus wieder miteinander verseilt sind.

Durch die Erfindung wird die Aufgabe gelöst, einen Lithotriptor der eingangs erwähnten Art so auszubilden, daß eine hohe Kraft auf das Fangkörbchen übertragen werden kann, um einerseits auch harte Konkremente ohne Gefahr eines Bruches von Teilen des Zugstranges oder der Fangstränge zerlegen zu können, andererseits aber die Möglichkeit besteht, das Fangkörbchen von sehr harten und damit unzertrümmerbaren Konkrementen nachträglich wieder zu lösen.

Dies wie erfindungsgemäß dadurch erreicht, daß das Drahtseil mindestens zweifach verseilt ist und die verformten Seillitzen an der Fangkörbchenspitze durch Löten oder Klemmen aneinander befestigt sind und daß die Zugvorrichtung zum Festklemmen des Drahtseils an dessen proximalem Ende ein Spannfutter aufweist, welches am hinteren Ende der Zugvorrichtung koaxial zu dem Drahtseil angeordnet ist und am Spannfuttergrund eine zentrale Öffnung für das Drahtseil aufweist.

Gemäß der Erfindung weist die Zugvorrichtung eine Klemmvorrichtung zum Festklemmen des Drahtseils an dessen proximalen Ende auf, wobei die Klemmvorrichtung als am hinteren Ende der Zugvorrichtung koaxial zu dem Zugstrang angeordnetes Spannfutter ausgebildet ist, welches am Spannfuttergrund eine zentrale Öffnung für den Zugstrang aufweist. Durch die Ausbildung des Zugstranges als verseiltes Drahtseil ist es möglich, den Zugstrang mit einem Spannfutter festzuklemmen. Im Gegensatz dazu wäre das gemeinsame Festklemmen mehrerer nicht miteinander verbundener und zueinander parallel verlaufender Seilstränge in einem Spannfutter nicht möglich, da sich hierbei die Klemmkräfte undefiniert auf die einzelnen Seilstränge verteilen würden und somit ein Festklemmen aller Seilstränge gemeinsam nicht gewährleistet wäre.

Das Spannfutter ist nach Art eines herkömmlichen Bohrfutters z.B. einer Handbohrmaschine ausgebildet. Als besonders vorteilhaft bei der Ausgestaltung der Klemmvorrichtung als Spannfutter hat sich herausgestellt, daß die Zugkräfte sehr gleichmäßig in den Zugstrang eingeleitet werden und keine wesentliche Schwächung oder gar eine Beschädigung des Zugstranges durch die Klemmkräfte hervorgerufen wird. Außerdem ist es möglich, den Zugstrang knickfrei an jeder Stelle desselben festzulegen, so daß ein entsprechend langer Zugstrang problemlos auch mit unterschiedlich langen Kathetern verwendet werden kann. Ferner besteht die Möglichkeit, den Zugstrang nach dem Einfangen des Konkrementes bei noch gelöstem Spannfutter zurückzuziehen, bis das Konkrement von den Fangsträngen fest umschlossen wird, und den Zugstrang erst dann in dem Spannfutter festzuklemmen. Hierdurch kann ggf. der Hub der Zugvorrichtung beträchtlich verkleinert werden.

Das Spannfutter kann vorteilhaft aus Edelstahl bestehen und über eine Metallhülse mit der Zugvorrichtung verbunden sein, die vorzugsweise aus Kunststoff besteht. Gemäß einer weiteren vorteilhaften Ausgestaltung der Erfindung ist die den Zugstrang klemmende Innenfläche des Spannfutters mit einer griffigen Struktur, z. B. einer Riffelung, versehen.

Durch die erfindungsgemäße Verwendung eines zweifach verseilten Drahtseils aus in sich verseilten Seillitzen, die ihrerseits miteinander zur Ausbildung des Drahtseils verseilt sind, weist der Zugstrang eine hohe Reißfestigkeit bei gleichmäßiger Spannungsverteilung über die Seillitzen hin auf. Durch die Ausbildung des Fangkörbchens aus verformten Seillitzen des Drahtseiles ist auch deren Reißfestigkeit verhältnismäßig hoch. Die schwächste Stelle des Lithotriptors liegt daher wegen der nachträglichen Befestigung der freien Enden der Seillitzen durch Löten oder Klemmen an der Fangkörbchenspitze, wodurch hier eine Art Sollbruchstelle ausgebildet ist, die bei sehr hoher Zugbelastung bricht. Dadurch öffnet sich das Fangkörbchen und gibt den eingefangenen, unzertrümmerbaren Stein frei, so daß der Lithotriptor aus der Körperhöhle herausgezogen werden kann. Durch die Ausbildung des Zugstranges als zweifach verseiltes Drahtseil und der Fangstränge des Fangkörbchens aus Seillitzen dieses Drahtseiles können im Notfall sehr hohe Zugkräfte auf die Befestigungsstelle der Fangstränge an der Fangkörbchenspitze übertragen werden, so daß deren Festigkeit groß genug sein kann, um sich bei normaler Anwendung des Lithotriptors nicht zu öffnen.

Gemäß einer bevorzugten Ausgestaltung der Erfindung sind die Seillitzen des Drahtseiles zwischen dem Fangkörbchenfuß, an dem sie von einer zweiten Hülse zusammengehalten werden, und einer im Abstand vom Fangkörbchenfuß angeordneten, das Drahtseil umgebenden ersten Hülse voneinander entseilt und verlaufen parallel zueinander. Dabei können die Litzenanzahl des Drahtseils die Anzahl der Fangstränge des Fangkörbchens übersteigen und diese übersteigende Anzahl von Seillitzen in der zweiten Hülse enden. Dadurch daß der Zugstrang an seinem Endabschnitt entseilt ist, wird dort seine Flexibilität erhöht, so daß das Fangkörbchen in der Körperhöhle leichter manipuliert werden kann, um das Konkrement einzufangen. Als besonders vorteilhaft hat sich dabei herausgestellt, wenn die Teilstrecke zwischen den Hülsen eine Länge von 3 bis 15 cm, vorzugsweise von 7 bis 11 cm hat.

Vorteilhaft weist das Drahtseil sieben Seillitzen und diese jeweils neunzehn Drähte auf.

Die Zugvorrichtung weist bevorzugt eine in einem Griffstück verschiebbare hohle Zugstange auf, die aus dem Griffstück herausragt und an der das Spannfutter festgelegt ist und die auf ihrem aus dem Griffstück herausragenden Abschnitt einen Außengewindeabschnitt aufweist, in welchen eine an dem Griffstück abgestützte Stellmutter eingreift, die mit einem Kegelritzel gekuppelt ist, welches mittels eines mit dem Kegelritzel kämmenden Kegelrades mit einer Betätigungshandhabe drehbar ist.

Die Zähnezahl des Kegelritzels und des Kegelrades können miteinander übereinstimmen. Jedoch kann die Zähnezahl des Kegelrades auch größer als diejenige des Kegelritzels sein, so daß eine Kraftübersetzung zum festen Anziehen der Stellmutter vorliegt. Die Handhabe kann beispielsweise eine an dem Kegelrad festgelegte Handkurbel sein, ist jedoch vorzugsweise als Handrad ausgebildet.

Die Erfindung wird nachfolgend anhand eines bevorzugten Ausführungsbeispiels unter Bezugnahme auf die Zeichnung erläutert.

In der Zeichnung zeigen:
- Fig. 1: den gesamten Endabschnitt des Zugstrangs und das Fangkörbchen des Lithotriptors in der Seitenansicht;
- Fig. 2: eine Seitenansicht eines ersten bevorzugten Ausführungsbeispiels des gesamten Lithotriptors mit Spannfutter und einer Kippmutter; und
- Fig. 3: eine Seitenansicht eines zweiten bevorzugten Ausführungsbeispiels des gesamten Lithotriptors mit Spannfutter und einem Schneckentrieb.

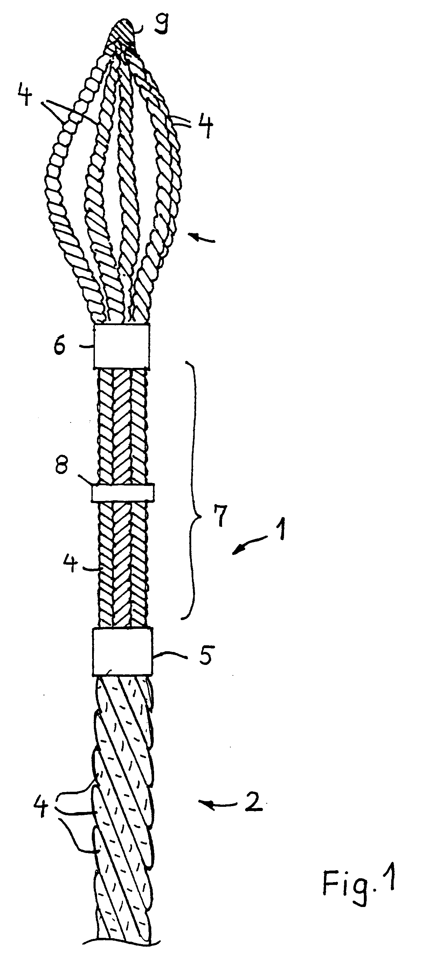

Wie aus Fig. 1 ersichtlich, ist der Zugstrang 1 des Lithotriptors über einen wesentlichen Längenbereich als zweifach verseiltes Drahtseil 2 ausgebildet und weist am distalen Ende ein Fangkörbchen 3 auf, das zumindest aus einer Teilanzahl von Seillitzen 4, die das Drahtseil 2 bilden, geformt wird. Das Drahtseil 2 verläuft vom proximalen Ende kommend bis zu einer ersten Hülse 5 zweifach verseilt. Im dargestellten Ausführungsbeispiel umfaßt das Drahtseil 2 sieben in sich verseilte Seillitzen 4, die aus jeweils neunzehn Drähten bestehen. Hinter der ersten Hülse 5 ist das Drahtseil 2 entseilt, d. h. die Seillitzen 4 laufen parallel zueinander bis zu einer zweiten Hülse 6 am Fangkörbchenfuß. Über diese entseilte Teilstrecke 7 hin zwischen erster Hülse 5 und zweiter Hülse 6 sind die Seillitzen 4 nicht vorgespannt, sondern gestreckt, so daß eine biegeweiche Teilstrecke 7 des Drahtseils 2 entsteht. Die Teilstrecke 7 ist ca. 3 bis 15 cm lang, vorzugsweise 7 bis 11 cm. Je nach Länge der Teilstrecke 7 kann es nützlich sein, eine oder mehrere Führungshülsen 8 vorzusehen, die verhindern, daß sich die Seillitzen 4 auf der Teilstrecke 7 aufsplitten und dadurch möglicherweise das Fangen des Konkrements erschweren.

Die erste Hülse 5 dient dazu, ein weiteres Entseilen des Drahtseils 2 in Richtung auf sein proximales Ende zu verhindern. Sowohl die erste Hülse 5 als auch die zweite Hülse 6 sollen deswegen Befestigungsvorrichtungen enthalten, die eine Fixierung der Seillitzen 4 gewährleisten. Dies kann sowohl über einen Klemmvorgang, als auch durch Löten, durch ein Klebemittel oder andere geeignete Befestigungen erfolgen. Hinter der zweiten Hülse 6 sind nur noch diejenigen Seillitzen 4 weitergeführt, die zur Bildung des Fangkörbchens 3 herangezogen werden. Im Ausführungsbeispiel sind es fünf Seillitzen 4, die nach dem Entseilen vom Drahtseil 2 nach außen konvex gebogen verformt wurden. Die das Fangkörbchen 3 bildenden Seillitzen 4 sind mit ihren freien Enden aneinander befestigt, im Ausführungsbeispiel in einer Kegelhülse 9 verlötet, von welcher die Fangkörbchenspitze gebildet wird. Es ist darauf zu achten, daß die Festigkeit der Verbindung der freien Litzenenden miteinander und mit der Kegelhülse 9 kleiner ist als die zulässige maximale Zugspannung der Seillitzen 4.

Zur Bildung des Fangkörbchens 3 können prinzipiell alle Seillitzen 4, bis auf die Seele des Drahtseils 2, herangezogen werden. Die Seele des Drahtseils 2 endet entweder an der ersten Hülse 5 oder an der zweiten Hülse 6. Alle übrigen nicht zur Bildung des Fangkörbchens 3 benötigten Seillitzen 4 enden an der zweiten Hülse 6, wo sie vorzugsweies verlötet sind.

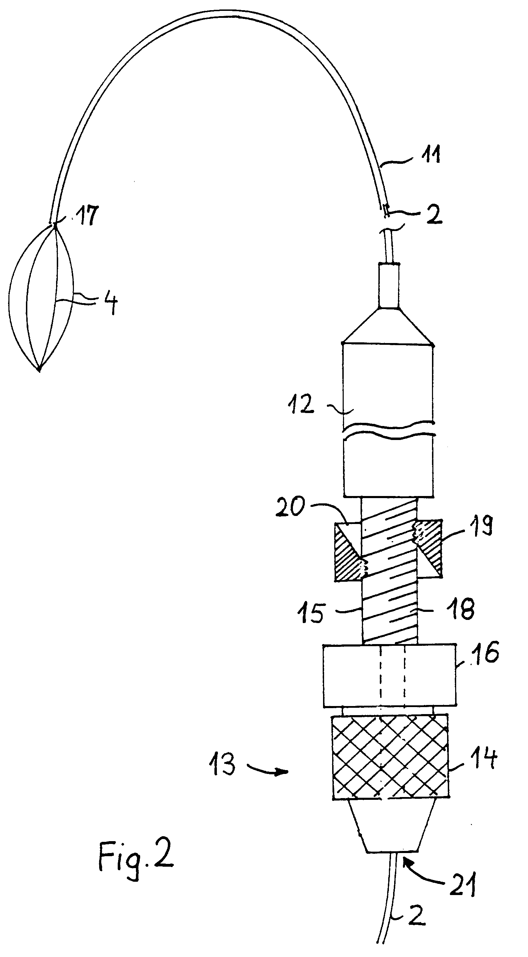

Fig. 2 zeigt eine Seitenansicht eines ersten bevorzugten Ausführungsbeispiels des gesamten Lithotriptors einschließlich einer Zugvorrichtung 10 zum Ein- und Ausfahren des Fangkörbchens 3 aus einer des Drahtseil 2 bowdenzugartig führenden Ummantelung 11. Diese flexible Ummantelung 11 besteht beispielsweise aus einem aus schraubenlinienförmig gewickeltem Stahldraht bestehenden Schlauch, der an einem Griffstück 12 abgestützt ist und durch welchen das Drahtseil 2 hindurch verläuft, an dessen distalem Ende das Fangkörbchen 3 angeordnet ist. Das proximale Ende des Drahtseils 2 ist mittels einer Klemmvorrichtung 13, die im wesentlichen aus einem Spannfutter 14 besteht, in einer hohlen Zugstange 15 festgelegt, welche in dem Griffstück 12 axial verschiebbar geführt ist. Als Übergangsteil zwischen der Zugstange 15, die vorzugsweise aus Kunststoff besteht, und der Klemmvorrichtung 13, die vorzugsweise aus Edelstahl wie z. B. V2A besteht, ist eine Metallhülse 16 vorgesehen. Durch Hineinschieben der Zugstange 15 in das Griffstück 12 wird das Fangkörbchen 3 von dem Zugstrang 1 aus dem distalen Ende 17 der Ummantelung 11 herausgefahren, wohingegen das Fangkörbchen 3 durch Herausziehen der Zugstange 15 aus dem Griffstück 12 in das distale Ende 17 des Katheters eingefahren wird.

Auf der Zugstange 15 ist ein Außengewinde 18 mit geringer Steigung ausgebildet, so daß eine Gewindestange vorliegt. Auf dieser ist eine Stellmutter 19 verschraubbar. Die Stellmutter 19 ist gemäß diesem Ausführungsbeispiel als Schnellmutter ausgebildet, die mit ihrem Innengewinde durch Verkippen auf der Zugstange 15 außer Eingriff mit dem Außengewinde 18 gebracht werden kann und in dieser Kipplage auf der Zugstange 15 axial frei verschiebbar ist. Hierzu weist die Stellmutter 19 eine schräg zu ihrer Gewindebohrung verlaufende Schrägbohrung 20 auf, deren Durchmesser zwecks Spiel und dadurch bedingter leichter Verschiebbarkeit der Stellmutter 19 nach dem Verkippen etwas größer als der Außendurchmesser des Außengewindes 18 der Gewinde- oder Zugstange 15 ist.

Das Spannfutter 14 ist über die Metallhülse 16 am freien Ende der Zugstange 15 angeordnet. Der Boden des Spannfutters 14 weist eine durchgehende, zentrale Öffnung 21 auf, durch die das Drahtseil 2 aus der hohlen Zugstange 15 herausgeführt ist. Die Klemmvorrichtung 13 ist so angeordnet, daß die Öffnung 21 mit der Längsachse der hohlen Zugstange 15 fluchtet.

Das Spannfutter 14 kann wie ein konventionelles Bohrfutter einer elektrischen Handbohrmaschine als Dreibackenfutter ausgebildet sein. Jedoch braucht der Verstellzahnkranz für das Öffnen und Schießen des Spannfutters 14 mittels eines Schlüssels bei der erfindungsgemäßen Vorrichtung nicht vorhanden zu sein, da ein manuelles Betätigen des Spannfutters 14 im vorliegenden Fall in aller Regel ausreicht.

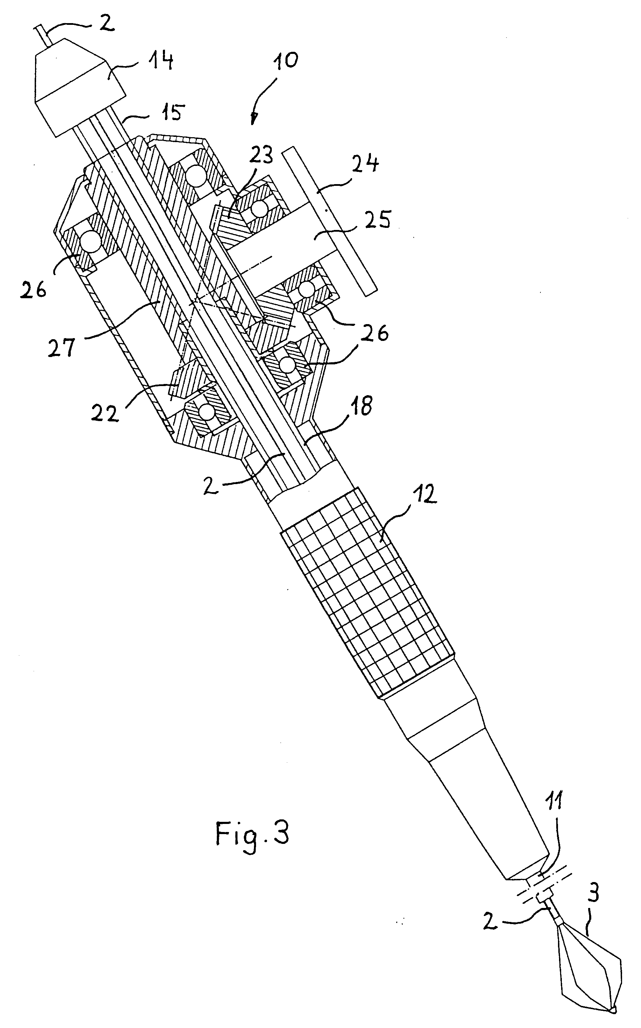

Fig. 3 zeigt eine teilweise geschnittene Längsansicht eines zweiten bevorzugten Ausführungsbeispiels des gesamten Lithotriptors. Im Gegensatz zu dem in Fig. 2 dargestellten ersten bevorzugten Ausführungsbeispiel wird die Stellmutter 27 der Zugvorrichtung 10 zwecks Einfahrens des Fangkörbchens 3 jedoch nicht durch Anfassen mit der Hand, sondern über ein Winkelgetriebe aus einem die hohle Zugstange 15 koaxial umgebenden, mit der Stellmutter 27 fest gekuppelten Kegelritzel 22 und einem mit diesem kämmenden Kegelrad 23 mittels eines Handrades 24 gedreht, das über eine dem Handrad 24 und dem Kegelzahnrad 23 gemeinsame Welle 25 mit diesem fest verbunden ist. Die hülsenförmige Stellmutter 27, das Kegelritzel 22 sowie das Kegelzahnrad 23 mit dem Handrad 24 sind in einem an das Griffstück 12 angebauten Gehäuse 27 in Kugellagern 26 gelagert.

## Patentansprüche

1. Lithotriptor mit einem Fangkörbchen (3), das von mehreren nach außen gebogen verlaufenden Fangsträngen aus Drahtmaterial gebildet wird und einen Fangkörbchenfuß sowie eine Fangkörbchenspitze aufweist, an der die Fangstränge aneinander befestigt sind, die an dem Fangkörbchenfuß mit einem aus mehreren Zugdrähten bestehenden Zugstrang (1) verbunden sind, welcher über einen verseilten Längenbereich als verseiltes Drahtseil (2) ausgebildet ist, in einer Ummantelung (11) geführt ist und von einer Zugvorrichtung (10) zum Ein- und Ausfahren des Fangkörbchens (3) in der Ummantelung (11) verschiebbar ist, wobei die Litzenanzahl des Drahtseils (2) mindestens der Anzahl der Fangstränge des Fangkörbchens (3) entspricht, die von verformten Seillitzen (4) des Drahtseils (2) gebildet werden, welche vom Drahtseil (2) aus bis zur Fangkörbchenspitze durchlaufen, dadurch gekennzeichnet, daß das Drahtseil (2) mindestens zweifach verseilt ist und die verformten Seillitzen (4) an der Fangkörbchenspitze nachträglich durch Löten oder Klemmen aneinander befestigt sind, und daß die Zugvorrichtung (10) zum Festklemmen des Drahtseils (2) an dessen proximalem Ende ein Spannfutter (14) aufweist, welches am hinteren Ende der Zugvorrichtung (10) koaxial zu dem Drahtseil (2) angeordnet ist und am Spannfuttergrund eine zentrale Öffnung (21) für das Drahtseil (2) aufweist.

2. Lithotriptor nach Anspruch 1, dadurch gekennzeichnet, daß das Spannfutter (14) als Dreibackenfutter ausgebildet ist.

3. Lithotriptor nach Anspruch 1 oder 2, dadurch gekennzeichnet, daß das Spannfutter (14) aus Edelstahl besteht und über eine Metallhülse (16) mit der Zugvorrichtung (10) verbunden ist, die vorzugsweise aus Kunststoff besteht.

4. Lithotriptor nach Anspruch 1, dadurch gekennzeichnet, daß die das Drahtseil (1) klemmende Innenfläche des Spannfutters (14) mit einer griffigen Struktur, z.B. einer Riffelung, versehen ist.

5. Lithotriptor nach Anspruch 1, dadurch gekennzeichnet, daß die Zugvorrichtung (10) eine in einem Griffstück (12) verschiebbare hohle Zugstange (15) aufweist, die aus dem Griffstück (12) herausragt und an der das Spannfutter (14) festgelegt ist und die auf ihrem aus dem Griffstück (12) herausragenden Abschnitt einen Außengewindeabschnitt (18) aufweist, in welchen eine an dem Griffstück (12) abgestützte Stellmutter (19) eingreift, die mit einer schräg zur Gewindeachse verlaufenden Schrägbohrung (20) etwas größeren Durchmessers als der Außendurchmesser des Außengewindeabschnitts (16) versehen ist und durch Verkippen auf der Zugstange (15) auf dieser axial verschiebbar ist.

6. Lithotriptor nach Anspruch 1, dadurch gekennzeichnet, daß die Zugvorrichtung (10) eine in einem Griffstück (12) verschiebbare hohle Zugstange (15) aufweist, die aus dem Griffstück (12) herausragt und an der das Spannfutter (14) festgelegt ist und die auf ihrem aus dem Griffstück (12) herausragenden Abschnitt einen Außengewindeabschnitt (18) aufweist, in welchen eine an dem Griffstück (12) abgestützte Stellmutter (27) eingreift, die mit einem Kegelritzel (22) gekuppelt ist, welches mittels eines mit dem Kegelritzel (22) kämmenden Kegelrades (23) mit einer Betätigungshandhabe (24) drehbar ist.

7. Lithotriptor nach Anspruch 6, dadurch gekennzeichnet, daß die Betätigungshandhabe als Handrad (24) ausgebildet ist.

8. Lithotriptor nach Anspruch 1, dadurch gekennzeichnet, daß die Seillitzen (4) des Drahtseils (2) von dem Fangkörbchenfuß aus über eine Teilstrecke (7) des Zugstrangs (1) hin voneinander entseilt sind und parallel zueinander verlaufen.

9. Lithotriptor nach Anspruch 8, dadurch gekennzeichnet, daß die Teilstrecke (7) eine Länge von 3 bis 15 cm, vorzugsweise von 7 bis 11 cm hat.

## Claims

1. Lithotriptor with a catch basket (3) which is formed by a plurality of outwardly curved catch strings made of wire material and which comprises a catch basket foot and a catch basket top at which the catch strings are fixed to each other, the catch strings being connected at the catch basket foot to a pull string (1) which consists of a plurality of pull wires, which is formed as a stranded wire rope (2) along a stranded length section and guided in a casing (11) and which is further slidable in the casing (11) by means of a pull mechanism (10) for introducing and retracting the catch basket (3), wherein the number of strands of the wire rope (2) corresponds to at least the number of catch strings of the catch basket (3), the catch strings are formed by deformed rope strands (4) of the wire rope (2) which run through from the wire rope (2) up to the catch basket top, characterized in that the wire rope (2) is stranded at least twice and the deformed rope strands (4) are fixed subsequently to each other at the catch basket top by soldering or clamping, and that the pull mechanism (10) comprises a chuck (14) for clamping the wire rope (2) at its proximal end, and the chuck (14) is arranged at the rear end of the pull mechanism (10) coaxially to the wire rope (2) and comprises at its base a central opening (21) for the wire rope (2).

2. Lithotriptor according to claim 1, characterized in that the chuck (14) is formed as a three cheek chuck.

3. Lithotriptor according to claim 1 or 2, characterized in that the chuck (14) consists of high-grade steel and is connected via a metal sleeve (16) to the pull mechanism (10) which is preferably made of plastics.

4. Lithotriptor according to claim 1, characterized in that the inner surface of the chuck (14) clamping the wire rope (2) is provided with a structure affording a firm grip, e.g. a corrugation pattern.

5. Lithotriptor according to claim 1, characterized in that the pull mechanism (10) comprises a hollow pull rod (15) which is slidable in and projects out of a handle (12) and on which the chuck (14) is secured and which further comprises an outside thread section (18) at its portion projecting out of the handle (12), and an adjusting screw nut (19) supported at the handle (12) engages the outside thread portion (18), the nut (19) being provided with a sloping bore (20) running oblique to the thread axis and having a diameter being slightly larger than the outer diameter of the outside thread section (18), wherein the nut (19) is axially slideable on the pull rod (15) after being canted on it.

6. Lithotriptor according to claim 1, characterized in that the pull mechanism (10) comprises a hollow pull rod (15) which is slidable in and projects out of a handle (12) and on which the chuck (14) is secured and which further comprises an outside thread section (18) at its portion projecting out of the handle (12), and an adjusting screw nut (27) supported at the handle (12) engages the outside thread portion (18), the nut (27) being coupled with a conical pinion (22) which is rotatable by means of a conical gear wheel (23) having an operation handle (24) and engaging the conical pinion (22).

7. Lithotriptor according to claim 6, characterized in that the operation handle is formed as a handwheel (24).

8. Lithotriptor according to claim 1, characterized in that the rope strands (4) of the wire rope (2) are unstranded and run parallel to each other from the foot of the catch basket along a part section (7) of the pull string (1).

9. Lithotriptor according to claim 8, characterized in that the part section (7) has a length of 3 to 15 cm, preferably of 7 to 11 cm.

## Revendications

1. Lithotripteur comprenant un petit panier de saisie (3) formé de plusieurs câblots de saisie s'étendant en courbe vers l'extérieur et formés de fils métalliques, présentant un pied de petit Panier de saisie ainsi qu'une tête de petit panier de saisie où les câblots de saisie sont solidarisés, ceux-ci étant liés, au pied de petit panier de saisie, à un câblot de manoeuvre (1) composé de plusieurs fils métalliques de manoeuvre, lequel forme, sur une portion longitudinale toronnée, un câble toronné en fils d'acier (2), est guidé à l'intérieur d'une gaine (11) et peut être coulissé à l'intérieur de la gaine (11) pour introduire et extraire le petit panier de saisie (3) dans la et de la gaine (11) au moyen d'un dispositif de manoeuvre (10), le nombre de torons du câble métallique (2) correspondant au moins au nombre de câblots de saisie du petit panier de saisie (3) qui sont constitués de torons de câble (4) déformés du câble métallique (2), lesquels s'étendent du câble métallique (2) à la tête de petit panier de saisie, caractérisé en ce que le câble métallique (2) est au moins doublement toronné et les torons de câble (4) déformés sont solidarisés postérieurement par soudage ou pincement à la tête de petit panier de saisie, et en ce que le dispositif de manoeuvre (10) présente pour le blocage du câble métallique (2) à l'extrémité proche de celui-ci, un mandrin de serrage (14) qui est disposé à l'extrémité arrière du dispositif de manoeuvre (10) coaxialement au câble métallique (2) et qui présente, à la base du mandrin de serrage, une ouverture centrale (21) pour le câble métallique (2).

2. Lithotripteur selon la revendication 1, caractérisé en ce que le mandrin de serrage (14) est un mandrin à trois mors.

3. Lithotripteur selon la revendication 1 ou 2, caractérisé en ce que le mandrin de serrage (14) est en acier spécial et est relié par l'intermédiaire d'une douille métallique (16) au dispositif de manoeuvre (10) qui est de préférence en matière plastique.

4. Lithotripteur selon la revendication 1, caractérisé en ce que la surface intérieure du mandrin de serrage (14) bloquant le câble métallique (1) est pourvue d'une structure de frottement, par exemple d'un striage.

5. Lithotripteur selon la revendication 1, caractérisé en ce que le dispositif de manoeuvre (10) présente une tige de manoeuvre creuse (15) coulissant à l'intérieur d'une poignée (12), faisant saillie hors de la poignée (12), à laquelle est fixé le mandrin de serrage (14) et qui présente sur sa partie faisant saillie hors de la poignée (12) une portion de filetage mâle (18) avec laquelle est en prise un écrou de manoeuvre (19) appuyé à la poignée (12) et pourvu d'un alésage incliné (20) s'étendant obliquement par rapport à l'axe du filetage et présentant un diamètre légèrement supérieur au diamètre extérieur de la portion de filetage mâle (16), lequel écrou de serrage (19) pouvant être déplacé axialement sur la tige de manoeuvre (15) après basculement sur celle-ci.

6. Lithotripteur selon la revendication 1, caractérisé en ce que le dispositif de manoeuvre (10) présente une tige de manoeuvre creuse (15) coulissant à l'intérieur d'une poignée (12), faisant saillie hors de la poignée (12), à laquelle est fixé le mandrin de serrage (14) et qui présente sur sa partie faisant saillie hors de la poignée (12), une portion de filetage mâle (18) avec laquelle est en prise un écrou de manoeuvre (27) appuyé à la poignée (12) et couplé avec un pignon conique (22) actionnable en rotation au moyen d'une manette d'actionnement (24) par l'intermédiaire d'une roue dentée conique (23) engrenant avec le pignon conique (22).

7. Lithotripteur selon la revendication 6, caractérisé en ce que la manette d'actionnement est un volant à main (24).

8. Lithotripteur selon la revendication 1, caractérisé en ce que les torons de câble (4) du câble métallique (2) s'étendant à partir du pied de petit panier de saisie sur une portion (7) du câblot de manoeuvre (1), sont détors et parallèles entre eux.

9. Lithotripteur selon la revendication 8, caractérisé en ce que la portion (7) a une longueur de 3 à 15 cm, de préférence de 7 à 11 cm.
